# EUROPEAN PATENT APPLICATION

(11) **EP 4 001 393 A1**
(43) Date of publication of application: **25.05.2022**
(21) Application number: 19938052.8
(22) Date of filing: 04.11.2019
(51) Int. Cl.: C12M 1/00, C12M 1/26, C12N 5/07, C12N 11/04

(54) **CELLULAR FIBERS, CELLULAR FIBER PRODUCTION SYSTEM, CELLULAR FIBER PRODUCTION METHOD, AND PROGRAM**

(30) Priority: 17.07.2019 JP 2019132257
(71) Applicant: Cellfiber Co., Ltd., Tokyo 113-8485 (JP)
(72) Inventor: ONOE Hiroaki, Tokyo 113-8485 (JP); IKEDA Kazuhiro, Tokyo 113-8485 (JP); SHOJI Kayoko, Tokyo 113-8485 (JP)
(74) Representative: Isarpatent
(86) International application number: PCT/JP2019/043176
(87) International publication number: WO 2021/009939

(57) **Abstract**

A cell fiber production system comprises: a first flow path through which a first fluid containing a cell flows; a second flow path for allowing a second fluid for preparing a hydrogel to converge around the first fluid in the first flow path; and an ejection port through which at least the first fluid and the second fluid are discharged together. The cell fiber production system is constructed that, at a start stage of production of a cell fiber, the first fluid is delivered after the second fluid reaches at least a convergence point between the first flow path and the second flow path.

## Description

### Technical field

The present invention relates to a cell fiber, a cell fiber production system, a cell fiber production method, and a program.

### Background Art

A method for culturing a cell on a two-dimensional surface is well known. Patent Literatures 1 and 2 below disclose, instead of such a method, a system for culturing and producing a cell in a hollow hydrogel fiber made of an alginate polymer. In this system, a cell solution containing cells is suspended in a hollow space of the alginate hydrogel fiber, and then the cell-containing hollow fiber is suspended in a cell culture medium. As a result, the cells are cultured in the hollow fiber. Patent Literature 1 describes that a wide variety of cells can be cultured at large scale levels.

### Citation List

### Patent Literature

Patent Literature 1: JP 2018-534936 A
Patent Literature 2: WO 2011/046105 A

### Summary

The inventor of the present application has found a problem that, when cells are cultured in a fiber as described in Patent Literature 1, the cells may leak from the fiber before or during the culture of the cells, depending on conditions for producing the cells and the fiber. Depending on the intended use of the produced cells, it is preferable to reduce an amount of cells leaking from the fiber and maintain the state in which the cells are confined in the fiber as much as possible.

Therefore, a cell fiber production system or a cell fiber production method with which it is possible to prevent leakage of cells from a fiber is desired.

According to an aspect, a cell fiber production system comprises: a first flow path through which a first fluid containing a cell flows; a second flow path for allowing a second fluid for preparing a hydrogel to converge around the first fluid in the first flow path; and an ejection port through which at least the first fluid and the second fluid are discharged together. The cell fiber production system is constructed that, at a start stage of production of a cell fiber, the first fluid is delivered after the second fluid reaches at least a convergence point between the first flow path and the second flow path.

According to another aspect, a cell fiber production method relates to a method using a nozzle that includes a first flow path through which a first fluid containing a cell flows, a second flow path for allowing a second fluid for preparing a hydrogel to flow along a flow of the first fluid around the first fluid, and an ejection port through which at least the first fluid and the second fluid are discharged together. The method comprises: delivering the second fluid at a start stage of production of a cell fiber; and delivering the first fluid after the second fluid reaches at least a convergence point between the first flow path and the second flow path.

According to another aspect, a program is a program for making computer execute the cell fiber production method according to said cell fiber production method.

### Advantageous Effects

According to the above aspects, the leakage of cells from the fiber can be prevented.

### Brief Description of Drawings

Fig. 1 is a schematic diagram illustrating an overall configuration of a cell fiber production system according to a first embodiment.
Fig. 2 is a diagram illustrating a flowchart of a cell fiber production method according to the first embodiment.
Fig. 3 is a graph showing changes over time in flow rates of a first fluid, a second fluid, a third fluid, and a cleaning fluid in the first embodiment.
Fig. 4 is a diagram illustrating a micrographic image of a cell fiber in one embodiment.
Fig. 5 is a diagram illustrating a flowchart of a cell fiber production method according to a second embodiment.
Fig. 6 is a graph showing changes over time in flow rates of a first fluid, a second fluid, and a cleaning fluid in the second embodiment.
Fig. 7 is a schematic diagram illustrating an overall configuration of a cell fiber production system according to a third embodiment.
Fig. 8 is a diagram illustrating a flowchart of a cell fiber production method according to the third embodiment.
Fig. 9 is a graph showing changes over time in flow rates of a first fluid, a second fluid, and a cleaning fluid in the third embodiment.
Fig. 10 is a diagram illustrating a micrographic image of a cell fiber according to a first example.
Fig. 11 is a diagram illustrating a micrographic image of a cell fiber according to a first reference example.

### Description of Embodiments

Embodiments will now be described with reference to the drawings. In the drawings below, the same or similar parts are designated by the same or similar reference numerals.

### [First Embodiment]

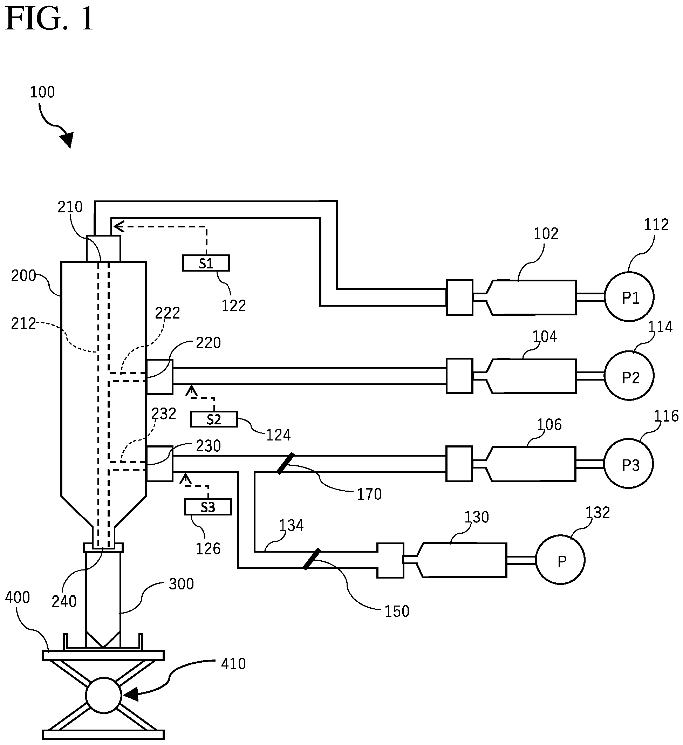

Fig. 1 is a schematic diagram illustrating an overall configuration of a cell fiber production system according to a first embodiment. The cell fiber production system 100 may have a first storage unit 102, a second storage unit 104, a third storage unit 106, a nozzle 200, and a reservoir 300.

The first storage unit 102 stores a first fluid containing cells. The first fluid may be, for example, a gel or liquid (suspension) containing cells. The type of gel or liquid is not particularly limited. The gel or liquid may be, for example, chitosan gel, collagen gel, gelatin, peptide gel, fibrin gel, laminin gel, nanocellulose, pullulan, dextran, culture medium or alginic acid, or a mixture thereof.

The type of cells contained in the first fluid is not particularly limited. Examples of such cells include pluripotent ES cells and iPS cells, various pluripotent stem cells (hematopoietic stem cells, neural stem cells, mesenchymal stem cells, etc.), and unipotent stem cells (such as hepatic stem cells, germ stem cells, etc.). Besides, the cells contained in the first fluid may be various differentiated cells including muscle cells such as skeletal muscle cells and myocardial cells, nerve cells such as cerebral cortex cells, fibroblasts, epithelial cells, hepatic cells, pancreatic beta cells, and skin cells. Furthermore, it is to be noted that the "cell" contained in the first fluid is not limited to a single cell, but includes a microorganism such as bacteria in addition to a cell tissue including multiple cells.

The first fluid may include a variety of growth factors suitable for cell culture, cell maintenance and proliferation, or expression of cell function, such as epithelial growth factor (EGF), platelet-derived growth factor (PDGF), transforming growth factor (TGF), insulin-like growth factor (IGF), fibroblast growth factor (FGF), or nerve growth factor (NGF).

The second storage unit 104 stores a second fluid for preparing a hydrogel (solution as a hydrogel precursor). The second fluid is preferably alginate or agarose. More specifically, the second fluid may be, for example, sodium alginate, potassium alginate, ammonium alginate, or a combination thereof. In addition, alginic acid may be a natural extract or a chemically modified alginic acid. Examples of the chemically modified alginic acid include methacrylate-modified alginic acid. Further, the second fluid may be a mixture of the above-mentioned alginate and agar, agarose, polyethylene glycol (PEG), polylactic acid (PLA), nanocellulose, or the like.

The third storage unit 106 stores a third fluid that gels the second fluid when it comes into contact with the second fluid. When the second fluid is the above-mentioned alginate polymer material, the third fluid may be a solution containing polyvalent cations. Examples of such a solution include a solution containing calcium ions or barium ions such as calcium chloride or barium chloride.

The nozzle 200 may have a first inlet 210, a second inlet 220, a third inlet 230, and an ejection port 240. The first inlet 210 is an inflow port for the first fluid and communicates fluidly with the first storage unit 102. The second inlet 220 is an inflow port for the second fluid and communicates fluidly with the second storage unit 104. The third inlet 230 is an inflow port for the third fluid and communicates fluidly with the third storage unit 106.

In the present specification, a flow path from the first inlet 210 or the first storage unit 102 to the ejection port 240 is referred to as a "first flow path". That is, the first flow path 212 may be defined by a flow path through which the first fluid containing cells passes.

In the present specification, a flow path from the second inlet 220 or the second storage unit 104 to a convergence point with the first flow path 212 is referred to as a "second flow path". That is, the second fluid joins the first flow path 212 through the second flow path 222 and then reaches the ejection port 240. Here, the convergence point between the first flow path 212 and the second flow path 222 is preferably located in the nozzle 200.

The second flow path 222 is formed to allow the second fluid for hydrogel preparation to converge around the first fluid in the first flow path 212. As a result, the second fluid flows around the flow of the first fluid along the direction of the flow of the first fluid. That is, the second fluid surrounds the first fluid in a cross section orthogonal to the flow of the first fluid. More specifically, it is preferable that the first fluid and the second fluid flow so as to form a laminar flow.

In the present specification, a flow path from the third inlet 230 or the third storage unit 106 to a convergence point with the first flow path 212 is referred to as a "third flow path". That is, the third fluid joins the first flow path 212 through the third flow path 232 and then reaches the ejection port 240. The third flow path 232 allows the third fluid to join the first flow path 212 on the downstream side with respect to the convergence point between the first flow path 212 and the second flow path 222. Here, the convergence point between the first flow path 212 and the third flow path 232 is preferably located in the nozzle 200.

The third fluid flows along the flow of the second fluid around the second fluid that surrounds the first fluid on the downstream side with respect to the convergence point between the first flow path 212 and the third flow path 232. The third fluid preferably forms a laminar flow together with the first fluid and the second fluid. The second fluid gels when coming into contact with the third fluid. Therefore, the second fluid ejected from the ejection port 240 is partially or completely gelled.

The sizes such as the flow path diameters of the first flow path 212, the second flow path 222, and the third flow path 232 can be appropriately designed according to the size of each part of the cell fiber to be produced.

It is preferable that the first flow path 212 extends in a direction substantially along gravity, at least on the downstream side with respect to the convergence point between the first flow path 212 and the second flow path 222. As a result, gravity is unlikely to be applied in the direction orthogonal to the flow direction of the first fluid, so that the second fluid flowing around the first fluid is easy to cover the first fluid with an even thickness in a cross section orthogonal to the flow direction of the first fluid.

The nozzle 200 is preferably configured to be replaceable with respect to the cell fiber production apparatus 100. As a result, when the flow path in the nozzle 200 is clogged, the clogging can be cleared by replacing the nozzle 200. The nozzle 200 particularly includes a narrow flow path and thus is more likely to be clogged than the rest of the apparatus. Therefore, when the nozzle 200 is configured to be replaceable, the cell fiber production apparatus 100 can be used for a long period of time.

The cell fiber production system 100 may have a first pump 112 for pumping the first fluid toward the ejection port 240, a second pump 114 for pumping the second fluid toward the ejection port 240, and a third pump 116 for pumping the third fluid toward the ejection port 240. The first pump 112, the second pump 114, and the third pump 116 may be configured to be capable of adjusting the flow rates and flow velocities of the first fluid, the second fluid, and the third fluid, respectively.

The flow rates of the first fluid, the second fluid, and the third fluid by the first pump 112, the second pump 114, and the third pump 116 may be adjustable by a user, or may be automatically adjusted by a preset program. In this case, the cell fiber production system 100 may have a control unit that controls pumps 112, 114, 116, and 132.

The cell fiber production system 100 may have a first sensor 122 that detects a delivery state of the first fluid, a second sensor 124 that detects a delivery state of the second fluid, and a third sensor 126 that detects a delivery state of the third fluid. The first sensor 122, the second sensor 124, and the third sensor 126 detect whether or not the first fluid, the second fluid, and the third fluid are flowing without causing a clog, respectively.

The cell fiber production system 100 may have a notification unit that notifies the user of various types of information. The notification unit may be, for example, a display, a light emitting unit, a buzzer, or the like. For example, when an abnormality such as clogging is detected in the flow path by the first sensor 122, the second sensor 124, and/or the third sensor 126, the notification unit notifies the user. Such notification may be a warning displayed on the display, a light emission or a sound indicating an abnormality, or the like.

The cell fiber production system 100 may have a reservoir 300 that receives an object ejected through the ejection port 240, and a stage 400 on which the reservoir 300 is placed. During the production of the cell fiber, the reservoir 300 preferably contains a solution suitable for receiving the cell fiber. Such a solution may be, for example, saline.

The cell fiber production system 100 preferably has an adjustment mechanism 410 that varies the distance between the reservoir 300 and the ejection port 240. In the present embodiment, the stage 400 is configured to be movable in the vertical direction. Alternatively, the nozzle 200 may be configured to be movable in the vertical direction.

The adjustment mechanism 410 allows the positional relationship between the ejection port 240 and the liquid level in the reservoir 300 to be freely changed during the production of the cell fiber. Here, during the production of the cell fiber, the ejection port 240 is preferably located below the liquid level in the reservoir 300, that is, in the liquid in the reservoir 300. As a result, the cell fiber ejected through the ejection port 240 is directly received in the liquid, so that damage to the cell fiber can be suppressed.

The cell fiber production system 100 may have a detector that can estimate or detect that the ejection port 240 is located in the liquid in the reservoir 300. Accordingly, the adjustment mechanism 410 can automatically change the distance between the reservoir and the ejection port so that the ejection port 240 is located in the liquid in the reservoir 300. The detector may be, for example, an imaging device (for example, a camera) provided in the cell fiber production system. The imaging device can detect the contact between the liquid level in the reservoir and the ejection port 240 of the nozzle.

The cell fiber production system 100 may have a cleaning mechanism for cleaning at least a part of the first flow path 212, the second flow path 222, and the third flow path 232. In the present embodiment, the cell fiber production system 100 may include a cleaning container 130 for storing a cleaning fluid and a cleaning pump 132 capable of pumping the cleaning fluid from the cleaning container 130. The cleaning fluid is preferably saline.

It is preferable that the cleaning container 130 fluidly communicates with at least one of the first flow path 212, the second flow path 222, and the third flow path 232 via a cleaning flow path 134. That is, the cleaning flow path 134 can allow the cleaning fluid to flow into at least one of the first flow path 212, the second flow path 222, and the third flow path 232. As a result, at least a part of the first flow path and the second flow path can be cleaned through the cleaning flow path.

The cleaning pump 132 may be configured to pump the cleaning fluid from the cleaning flow path 134 to the ejection port 240. As a result, the cleaning fluid can flow from the cleaning flow path 134 to the ejection port 240 via the first flow path 212, the second flow path 222, and/or the third flow path 232. Therefore, clogging in the flow paths 212, 222, and 232 can be prevented. In addition, this configuration provides an advantage that the flow paths 212, 222, and 232 can be cleaned without disassembling the cell fiber production system 100.

It is preferable that the cleaning flow path 134 communicates with a position in which the cleaning fluid can be supplied to a portion where clogging is likely to occur in the first flow path 212, the second flow path 222, and the third flow path 232. In the embodiment shown in Fig. 1, the cleaning flow path 134 is configured to allow the cleaning fluid to flow into the third flow path 232. That is, the cleaning fluid reaches the ejection port 240 from the cleaning container 130 via the cleaning flow path 134 and the third flow path 232.

In this case, the cleaning fluid can effectively clean at least a part of the third flow path 232, particularly near the convergence point between the first flow path 212 and the third flow path 232, via the cleaning flow path 134. The third flow path 232 is a flow path for the third fluid that gels the second fluid. If the second fluid accidentally flows into the third flow path 232, the second fluid will gel in the third flow path 232. Therefore, clogging is likely to occur especially in the vicinity of the convergence point between the first flow path 212 and the third flow path 232. In the present embodiment, the cleaning flow path 134 communicates with the third flow path 232, and thus, clogging can be prevented or eliminated by the cleaning fluid, particularly near the convergence point between the first flow path 212 and the third flow path 232.

In the embodiment shown in Fig. 1, the cleaning flow path 134 communicating with the cleaning container 130 communicates with the third flow path 232. Alternatively, the cleaning flow path 134 communicating with the cleaning container 130 may communicate with the first flow path 212 and/or the second flow path 222. The cleaning flow path 134 only have to be configured so that the cleaning fluid flows through a place of the flow path where clogging is likely to occur. Further, the cleaning flow path 134 communicating with the cleaning container 130 is not limited to communicating with one of the first flow path 212, the second flow path 222, and the third flow path 232, and may communicate with two or more of them.

It is preferable that a valve 150 is provided in the cleaning flow path 134. The valve 150 may be, for example, an on-off valve or a one-way valve. With this configuration, the cleaning flow path 134 is closed by the valve 150, at least during a period in which the cleaning fluid is not delivered. Therefore, it is possible to prevent the first fluid, the second fluid, and/or the third fluid from flowing back into the cleaning flow path 134.

In particular, in the embodiment shown in Fig. 1, it is possible to prevent the third fluid from flowing back into the cleaning flow path 134 during the period in which the cleaning fluid is not delivered.

As shown in Fig. 1, a valve 170 may be provided in the third flow path 232. The valve 170 may be, for example, an on-off valve or a one-way valve. This prevents the third fluid from flowing out to the flow path 232 and the flow path 212 even after the third pump 116 is stopped, whereby clogging due to gelation of the second fluid can be prevented at a portion of the third flow path 232 in the vicinity of the convergence point between the first flow path 212 and the third flow path 232. The valve 170 may be used in place of or together with the cleaning mechanism described above. However, the valve 170 is not necessary and may not be provided if it is unnecessary.

### [Cell fiber production method]

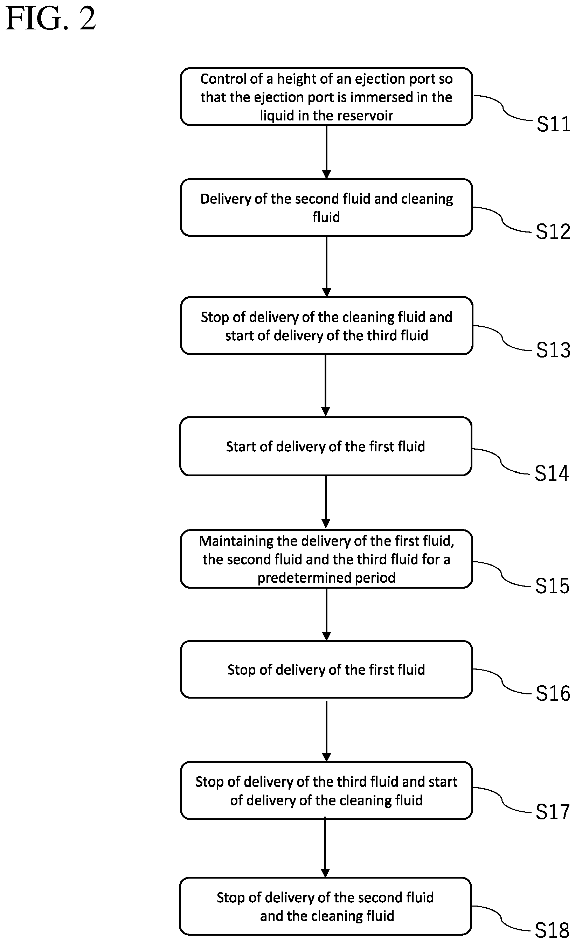

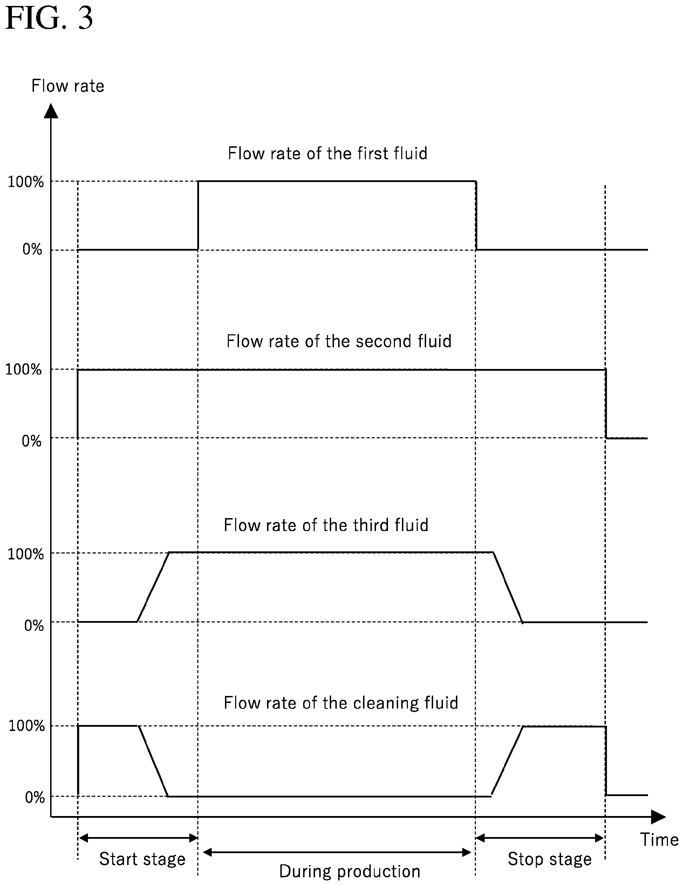

Fig. 2 is a diagram showing a flowchart of the cell fiber production method according to the first embodiment. Fig. 3 is a graph showing changes over time in flow rates of the first fluid, the second fluid, the third fluid, and the cleaning fluid during a period from the start of the cell fiber production to the end of the production. It is to be noted that the vertical axis in Fig. 3 shows a relative value of the flow rate. The cell fiber production method in the first embodiment can be implemented using the cell fiber production system 100 described above.

First, the reservoir 300 containing a liquid such as saline is placed on the stage 400 of the cell fiber production system 100, and the distance between the ejection port 240 of the nozzle 200 and the reservoir 300 is adjusted (step S11). Here, the height of the stage 400 is adjusted so that the ejection port 240 of the nozzle 200 is immersed in the liquid in the reservoir 300.

Here, it is preferable that the cell fiber production system 100 automatically estimates or detects that the ejection port 240 is located in the liquid in the reservoir 300, and the adjustment mechanism 410 automatically varies the distance between the reservoir 300 and the ejection port 240. However, the user may manually set the distance between the reservoir 300 and the ejection port 240.

Next, at a start stage of the production of the cell fiber, the second fluid for hydrogel preparation and the cleaning fluid are delivered toward the ejection port 240 (step S12). The second fluid can be delivered by the second pump 114. The cleaning fluid can be delivered by the cleaning pump 132. During delivery, it is preferable to form a laminar flow with the first fluid and the cleaning fluid, as will be described later.

The cleaning fluid may be delivered before the delivery of the second fluid for hydrogel preparation, or may be delivered at the same time as the delivery of the second fluid for hydrogel preparation. If the second fluid and the cleaning fluid are delivered at the same time, the period of the start stage (preparation stage) of the production of the cell fiber can be reduced.

Next, after the second fluid reaches at least the convergence point between the first flow path and the second flow path, preferably the ejection port 240, the delivery of the cleaning fluid is stopped and the delivery of the third fluid is started (step S13). The third fluid can be delivered by the third pump 116. It does not matter which of the timing of stopping the delivery of the cleaning fluid and the timing of starting the delivery of the third fluid comes first.

However, it is preferable that the delivery of the cleaning fluid is stopped at the same time as the delivery of the third fluid is started. This reduces the time required for the process of stopping the delivery of the cleaning fluid and starting the delivery of the third fluid.

More preferably, the flow rate of the third fluid is gradually increased while gradually lowering the flow rate of the cleaning fluid. As a result, it is possible to prevent the second fluid from entering the third flow path 232 in the process of stopping the delivery of the cleaning fluid and starting the delivery of the third fluid. Further, in this case, it is more preferable to form a laminar flow by the second fluid and the cleaning fluid, and then gradually increase the flow rate of the third fluid while gradually reducing the flow rate of the cleaning fluid. With this configuration, the flow of the cleaning fluid can be replaced with the flow of the third fluid with the state in which the laminar flow is formed being maintained. Therefore, a possibility that the second fluid and the third fluid are mixed by a turbulent flow in the process of starting the delivery of the third fluid is reduced, and a possibility that the flow path in the nozzle 200 is clogged by the gelled second fluid is further reduced.

In step S13, it is preferable to deliver the third fluid after the second fluid has reached at least the convergence point between the third flow path 232 and the first flow path 212, especially the ejection port 240.

Next, at the start stage of the production of the cell fiber, the delivery of the first fluid is started after both the second fluid and the third fluid reach at least the convergence point between the third flow path 232 and the first flow path 212, preferably the ejection port 240 (step S14). The first fluid can be delivered by the first pump 112.

As a result, the first fluid is delivered after the second fluid and the third fluid join. That is, after the second fluid is partially or completely gelled by the third fluid, the first fluid containing cells starts to join the second and third fluids. In other words, the first fluid containing cells is delivered after the front end of the cell fiber is partially or completely gelled. This makes it possible to further prevent cells from leaking from the front end of the fiber. In particular, even when the cells are cultured in the cell fiber, leakage of cells from the front end of the cell fiber can be prevented.

Next, the delivery of the first fluid, the second fluid, and the third fluid is maintained for a predetermined period (step S15). During this period, an object produced by the convergence of the first fluid, the second fluid, and the third fluid is ejected through the ejection port 240. Specifically, the ejected object is a cell fiber containing a substantially tubular hydrogel generated by the convergence of the second fluid and the third fluid, and the cell-containing first fluid added in the hydrogel. The length of the cell fiber can be adjusted according to the length of the delivery period in step S15.

At a stop stage of the production of the cell fiber, first, the delivery of the first fluid is stopped (step S16). It is preferable that, even after the delivery of the first fluid is stopped, the delivery of the second fluid and the delivery of the third fluid are continued for a predetermined period. This ensures that the rear end of the cell fiber is sufficiently closed with the (gelled) second fluid. Thus, leakage of cells in the cell fiber from the rear end of the fiber can be prevented.

Next, at the stop stage of the production of the cell fiber, the delivery of the third fluid is stopped and the delivery of the cleaning fluid is started (step S17). It is preferable that the delivery of the cleaning fluid is stopped at the same time as the start of the delivery of the third fluid. As a result, the fluid flowing in the nozzle 200 is gradually replaced with the cleaning fluid from the third fluid. Thus, the third fluid in the nozzle 200 is expelled through the ejection port 240 by the cleaning fluid. Accordingly, the second fluid does not come into contact with the third fluid, so that the gelation of the second fluid is suppressed, and the occurrence of clogging at the stop stage can be suppressed.

Next, at the stop stage of the production of the cell fiber, the delivery of the second fluid and the delivery of the cleaning fluid are stopped (step S18). The delivery of the cleaning fluid may be stopped before or after the delivery of the second fluid is stopped. Further, the delivery of the cleaning fluid may be stopped at the same time as the stop of the delivery of the second fluid.

Preferably, the delivery of the cleaning fluid is stopped simultaneous with or after the stop of the delivery of the second fluid. As a result, it is possible to prevent the second fluid from flowing back into the third flow path 232.

In the method described above, the delivery of the second fluid is stopped after the delivery of the third fluid is stopped at the stop stage of the production of the cell fiber. In this case, there is a period in which the second fluid flows even after the third fluid that gels the second fluid is stopped. Therefore, there is a period in which the second fluid expels the residue of the third fluid through the ejection port 240. Accordingly, it is possible to prevent the gelled second fluid from staying in the nozzle 200 and causing clogging after the production of the cell fiber is stopped.

After steps S11 to S18 described above, the cell fiber produced in the reservoir 300 may be transferred into a culture solution. In this case, the cells can be easily transferred into the culture solution, because the cells are confined in the cell fiber. In addition, since the cells are less likely to be mixed in a waste liquid in the reservoir 300, the waste liquid can be easily treated.

The cells in the cell fiber may be cultured for a predetermined period of time in a state of being transferred to the culture solution. Both ends of the fiber in the direction of extension of the fiber are closed with the hydrogel, so that the cells remain confined in the cell fiber even when the cells increase.

Whether or not the cleaning fluid, the second fluid, and/or the third fluid has reached an optional position in the flow path or the ejection port 240 at the start stage or the stop stage of the production of cell fiber can be determined artificially by the human eye or automatically by the cell fiber production system. For example, the cell fiber production system is equipped with a camera, and detects whether or not the cleaning fluid, the second fluid, and/or the third fluid is ejected through the ejection port 240 using the camera. With this configuration, the cell fiber production system can automatically determine whether or not the cleaning fluid, the second fluid, and/or the third fluid is ejected through the ejection port 240. Alternatively, the cell fiber production system may use the first sensor 122, the second sensor 124, and the third sensor 126 described above to determine whether or not the cleaning fluid, the second fluid, and/or the third fluid has reached the optional position in the flow path or the ejection port 240. In this case, it is only sufficient that the cell fiber production system estimates the time until the fluids reach the optional position in the flow path or the ejection port 240 from the sensors 122, 124, and 126 on the basis of the distance between the sensors 122, 124, and 126 and the optional position in the flow path or the ejection port 240 and flow velocities of the fluids. With this configuration, the cell fiber production system can automatically determine whether or not the cleaning fluid, the second fluid, and/or the third fluid has reached the optional position in the flow path or the ejection port 240 using the first sensor 122, the second sensor 124, and the third sensor 126.

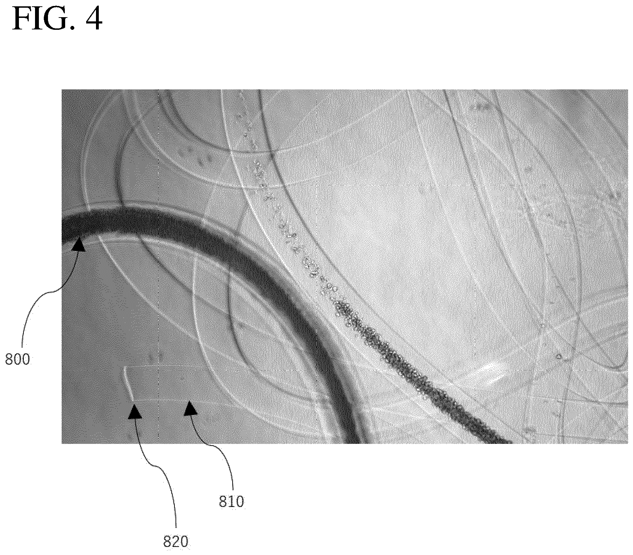

Fig. 4 is a diagram illustrating a micrographic image of a cell fiber in one embodiment. Fig. 4 is a micrographic image of a cell fiber produced by the above-mentioned device and method. Fig. 4 shows the state after cells are cultured in the cell fiber for a long time.

The cell fiber includes a plurality of cells 800 aligned along the fiber and a hydrogel 810 that wraps around the cells in a cross section that intersects the direction of extension of the fiber. The hydrogel 810 is closed at both ends 820 in the direction of extension of the fiber so as to confine the cells. The hydrogel 810 is composed of the second fluid gelled by the third fluid described above. In the example shown in Fig. 4, sodium alginate is used as the second fluid and calcium chloride is used as the third fluid. Further, in the example shown in Fig. 4, the cells in the first fluid are cardiomyocytes derived from iPS cells.

The outer diameter of the cell fiber is not particularly limited, but may be in a range of, for example, about 1 µm to 5 mm. The length of the cell fiber is not particularly limited. The length of the cell fiber may be, for example, about 20 mm to 100 cm. The inner diameter of the hydrogel (outer shell) constituting the cell fiber is not particularly limited, but may be in a range of, for example, about 100 nm to 1000 µm. Further, it is preferable that the length of the region of the gel portion containing no cells at both ends in the direction of extension of the cell fiber is, for example, 10 times or more the outer shape of the cell fiber. For example, the length of the region of the gel portion containing no cells at both ends of the cell fiber in the direction of extension of the cell fiber may be larger than 1 mm, preferably larger than 5 mm, and more preferably larger than 20 mm. This makes it possible to prevent the cells in the fiber from leaking from the end of the fiber.

### [Second Embodiment]

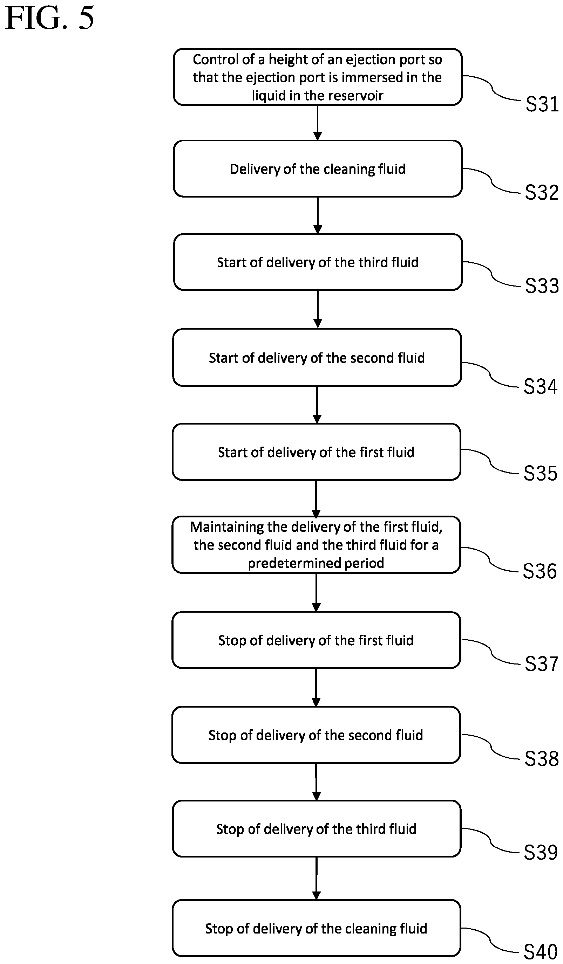

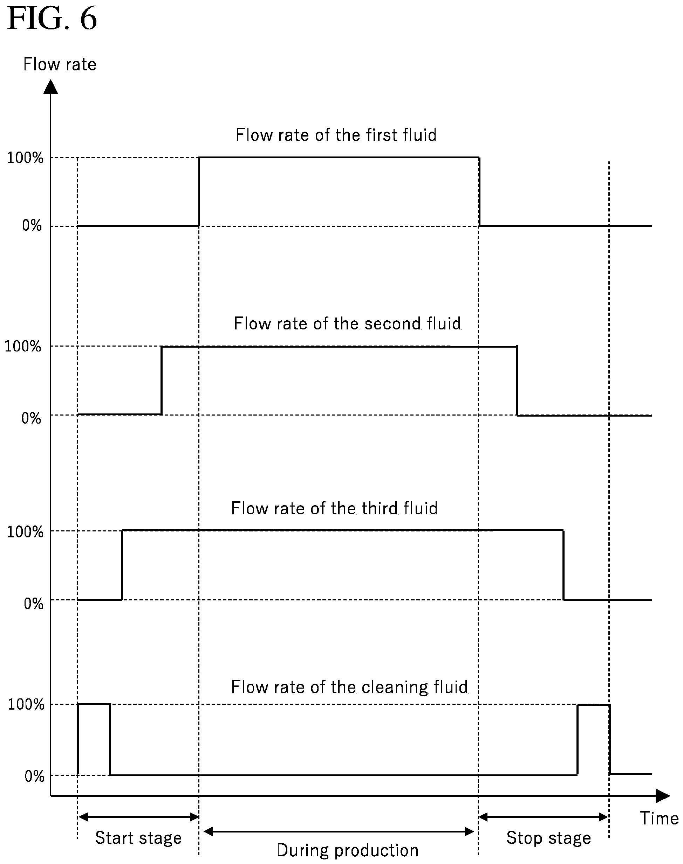

Fig. 5 is a diagram showing a flowchart of a cell fiber production method according to a second embodiment. Fig. 6 is a graph showing changes over time in flow rates of a first fluid, a second fluid, a third fluid, and a cleaning fluid during a period from the start of the cell fiber production to the end of the production. It is to be noted that the vertical axis in Fig. 6 shows a relative value of the flow rates. The cell fiber production method in the second embodiment can be implemented using the cell fiber production system 100 described in the first embodiment (see also Fig. 1).

First, the reservoir 300 containing a liquid such as saline is placed on the stage 400 of the cell fiber production system 100, and the distance between the ejection port 240 of the nozzle 200 and the reservoir 300 is adjusted (step S31). Step S31 is similar to step S11 in the first embodiment.

Next, at a start stage of production of the cell fiber, the cleaning fluid is delivered toward the ejection port 240 as needed (step S32). The cleaning fluid can be delivered by the cleaning pump 132.

Delivering the cleaning fluid before the delivery of the first fluid, the second fluid, or the third fluid can expel something remaining in the flow path. In particular, when the third fluid flows in while the second fluid remains in the flow path in the nozzle 200, the second fluid may gel and cause clogging. Delivering the cleaning fluid first can reduce a possibility of clogging described above.

Next, the delivery of the cleaning fluid is stopped, and the third fluid for gelling the second fluid is delivered toward the ejection port 240 (step S33). The third fluid can be delivered by the third pump 116.

Next, the second fluid for hydrogel preparation is delivered toward the ejection port 240 (step S34). The second fluid can be delivered by the second pump 114. The delivery of the second fluid is started after the third fluid has reached at least the convergence point between the third flow path 232 and the second flow path 222, preferably the ejection port 240. More preferably, the delivery of the second fluid is started after the flow of the third fluid is stabilized. In step S34, it is preferable that a laminar flow is formed by the second fluid and the third fluid.

Next, at the start stage of the production of the cell fiber, the delivery of the first fluid is started after both the second fluid and the third fluid reach at least the convergence point between the third flow path 232 and the first flow path 212, preferably the ejection port 240 (step S35). The first fluid can be delivered by the first pump 112.

As a result, the first fluid is delivered after the second fluid and the third fluid join. That is, after the second fluid is partially or completely gelled by the third fluid, the first fluid containing cells starts to join the second and third fluids.

Next, the delivery of the first fluid, the second fluid, and the third fluid is maintained for a predetermined period (step S36). Step S36 is similar to step S15 in the first embodiment.

Next, at a stop stage of the production of the cell fiber, first, the delivery of the first fluid is stopped (step S37). It is preferable that, even after the delivery of the first fluid is stopped, the delivery of the second fluid and the delivery of the third fluid are continued for a predetermined period. This ensures that the rear end of the cell fiber is sufficiently closed with the (gelled) second fluid. Thus, leakage of cells in the cell fiber from the rear end of the fiber can be prevented.

Next, at the stop stage of the production of the cell fiber, the delivery of the second fluid is stopped (step S38). Next, the delivery of the third fluid is stopped (step S39). Alternatively, the delivery of the second fluid may be stopped after the delivery of the third fluid is stopped.

Next, the cleaning fluid is delivered toward the ejection port 240 (step S40). Delivering the cleaning fluid after the delivery of the second fluid and the third fluid is stopped can prevent residues from remaining in the flow path in the nozzle 200.

In the second embodiment, at the start stage of the production, the delivery of the second fluid is started after the start of the delivery of the third fluid. Alternatively, at the start stage of the production, the delivery of the second fluid and the delivery of the third fluid may be started at substantially the same timing.

### [Third Embodiment]

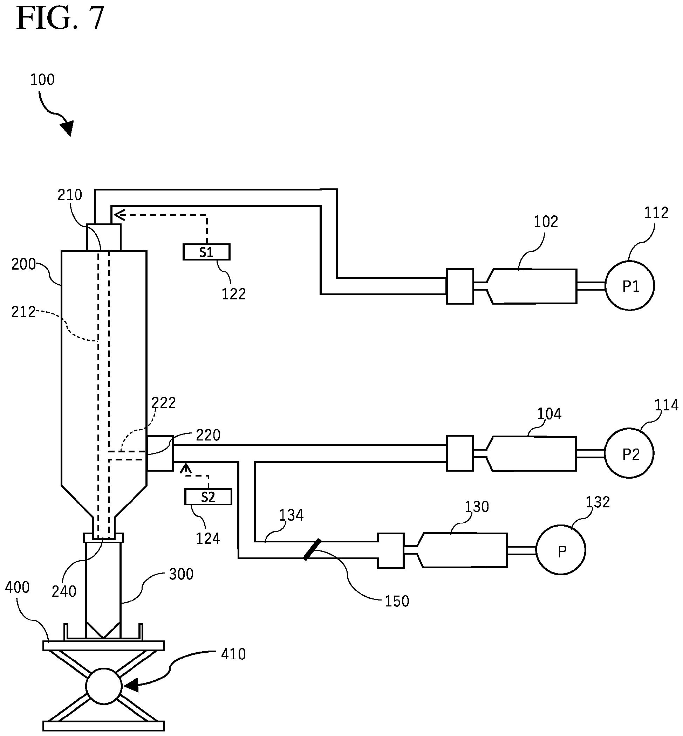

Fig. 7 is a schematic diagram illustrating an overall configuration of a cell fiber production system according to a third embodiment. In Fig. 7, the same reference numerals are given to the same configurations as those in the first embodiment. Further, the description of the configuration similar to that of the first embodiment may be omitted.

A cell fiber production system 100 may have a first storage unit 102, a second storage unit 104, a nozzle 200, and a reservoir 300. A first fluid stored in the first storage unit 102 is similar to that in the first embodiment. A second fluid stored in the second storage unit 104 is similar to that in the first embodiment.

In the third embodiment, the cell fiber production system 100 does not include the third storage unit 106 and the third flow path 232. A third fluid which gels the second fluid is placed in the reservoir 300 at the stage of producing the cell fiber. Note that the material of the third fluid may be similar to that of the first embodiment.

In the third embodiment, the first fluid and the second fluid are ejected together into the third fluid in the reservoir 300 through the ejection port 240. As a result, the second fluid comes into contact with the third fluid and gels in the reservoir 300.

The cell fiber production system 100 may have a cleaning mechanism for cleaning at least a part of the first flow path 212 and the second flow path 222. In the third embodiment, the cleaning flow path 134 communicates with the second flow path 222 so as to allow the cleaning fluid to flow into the second flow path 222. Alternatively, the cleaning flow path 134 may communicate with the first flow path 212 so as to allow the cleaning fluid to flow into the first flow path 212.

### [Cell fiber production method]

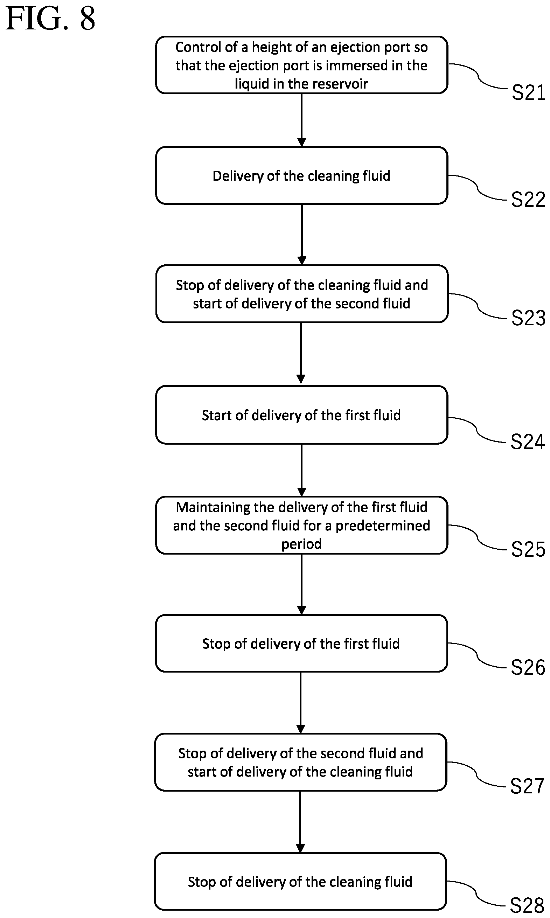

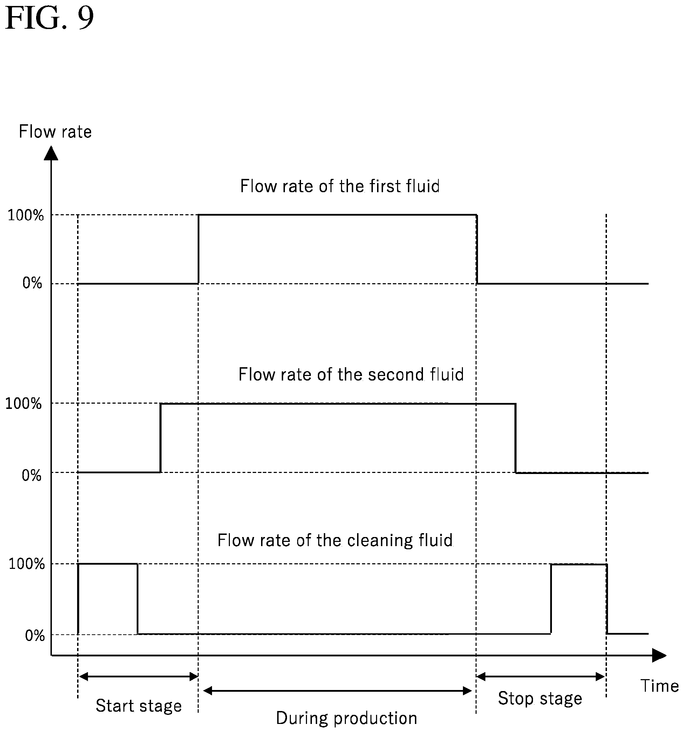

Fig. 8 is a diagram showing a flowchart of the cell fiber production method according to the third embodiment. Fig. 9 is a graph showing changes over time in flow rates of the first fluid, the second fluid, and the cleaning fluid during a period from the start of the cell fiber production to the end of the production. It is to be noted that the vertical axis in Fig. 9 shows a relative value of the flow rates. The cell fiber production method in the third embodiment can be implemented using the cell fiber production system 100 illustrated in Fig. 7.

First, the reservoir 300 containing a liquid such as saline is placed on the stage 400 of the cell fiber production system 100, and the distance between the ejection port 240 of the nozzle 200 and the reservoir 300 is adjusted (step S21). Here, the height of the stage 400 is adjusted so that the ejection port 240 of the nozzle 200 is immersed in the third fluid in the reservoir 300.

Here, it is preferable that the cell fiber production system 100 automatically estimates or detects that the ejection port 240 is located in the third fluid in the reservoir 300, and the adjustment mechanism 410 automatically varies the distance between the reservoir 300 and the ejection port 240. However, the user may manually set the distance between the reservoir 300 and the ejection port 240.

Next, at a start stage of production of the cell fiber, the cleaning fluid is delivered toward the ejection port 240 (step S22). Next, the delivery of the cleaning fluid is stopped, and the second fluid for hydrogel preparation is delivered toward the ejection port 240 (step S23) .

Next, after the second fluid reaches at least the convergence point between the first flow path and the second flow path, preferably the ejection port 240, the delivery of the first fluid is started (step S24). During delivery, it is preferable to form a laminar flow by the first fluid and the second fluid. Since the first fluid containing cells is delivered after the second fluid, the front end of the cell fiber is sufficiently closed by the (gelled) second fluid. Thus, leakage of cells in the cell fiber from the front end of the fiber during the production of the cell fiber can be prevented.

Next, the delivery of the first fluid and the second fluid is maintained for a predetermined period (step S25). During this period, an object produced by the convergence of the first fluid and the second fluid is ejected through the ejection port 240. The second fluid contacts the third fluid in the reservoir 300 and gels. Thus, a cell fiber containing a substantially tubular hydrogel and the cell-containing first fluid added in the hydrogel is produced. The length of the cell fiber can be adjusted according to the length of the delivery period in step S25.

At a stop stage of the production of the cell fiber, first, the delivery of the first fluid is stopped (step S26). It is preferable that, even after the delivery of the first fluid is stopped, the delivery of the second fluid is continued for a predetermined period. This ensures that the rear end of the cell fiber is sufficiently closed with the (gelled) second fluid. Thus, leakage of the cells in the cell fiber from the rear end of the fiber can be prevented.

Next, at the stop stage of the production of the cell fiber, the delivery of the second fluid is stopped and the delivery of the cleaning fluid is started (step S27). As a result, the inside of the nozzle 200 can be cleaned with the cleaning fluid at the stop stage of the production of the cell fiber.

Next, at the stop stage of the production of the cell fiber, the delivery of the cleaning fluid is stopped (step S28). After steps S21 to S28 described above, the cell fiber produced in the reservoir 300 may be transferred into a culture solution, as in the first embodiment.

Next, cell fibers according to a first example and a first reference example will be described. Note that the description of the following example and reference example does not intend to limit the invention, and the technical scope of the present invention is defined only by the invention-specifying matters set forth in claims that are appropriate from the above description.

The cell fiber according to the first example was produced by delivering the first fluid, the second fluid, and the third fluid according to the procedure described in the second embodiment using the apparatus illustrated in Fig. 1. More specifically, at the start stage of production, the delivery of the third fluid was started, and after three seconds, it was confirmed that the third fluid completely passed through the convergence point between the second flow path and the third flow path. Thereafter, the delivery of the second fluid was started. Next, when three seconds have elapsed after the start of the delivery of the second fluid, it was confirmed that the second fluid completely passed through the convergence point between the first flow path and the second flow path. Then, the delivery of the first fluid was started. In addition, at an end stage of the production, the first fluid, the second fluid, and the third fluid were stopped in this order.

Further, in the reference example, the order of starting the delivery of the first fluid and the second fluid and the order of stopping the delivery of the first fluid and the second fluid were reversed to the orders in the first example. Specifically, at the start stage of production, the delivery of the first fluid was started, and after three seconds, it was confirmed that the first fluid completely passed through the convergence point between the first flow path and the second flow path. Thereafter, the delivery of the second fluid was started. Next, when three seconds have elapsed after the start of the delivery of the second fluid, it was confirmed that the second fluid completely passed through the convergence point between the second flow path and the third flow path. Then, the delivery of the third fluid was started. In addition, at an end stage of the production, the third fluid, the second fluid, and the first fluid were stopped in this order.

The materials of the first fluid, the second fluid, and the third fluid for each of the first example and the first reference example are as follows.
- First fluid: Methyl cellulose suspension containing CHO DP 12 cells in a concentration of 10⁸ cells/mL
- Second fluid: Saline containing sodium alginate in a concentration of 1% (weight percent)
- Third fluid: Aqueous solution containing calcium chloride in a concentration of 100 mol/L and 3% by weight of sucrose

The flow velocities of the first fluid, the second fluid, and the third fluid during the production of the cell fibers are 50, 200, and 3600 µL/min, respectively.

The first fluid, the second fluid, and the third fluid were injected into the reservoir storing the saline, and after the first fluid, the second fluid, and the third fluid were stopped, the cell fibers were transferred to a medium, and the cells were cultured in the cell fibers.

Under the above conditions, five cell fibers were produced in each of the first example and the first reference example. The evaluation items (1) to (4) described later were evaluated. Table 1 below shows the evaluation results.

**[Table 1]**

| Evaluation | Example | Comparative example |
|---|---|---|
| (1) Confinement of cells at the end of the cell fiber immediately after the cell fiber is formed. | 5/5 | 0/5 |
| (2) Prevention of mixture of cells with the hydrogel immediately after the cell fiber is formed. | 5/5 | 1/5 |
| (3) Prevention of a leakage of cells while being cultured in the fiber. | 4/5 | 1/5 |

Regarding fractions in Table 1, the denominators indicate the number of samples, and the numerators indicate the number of samples that were evaluated well.

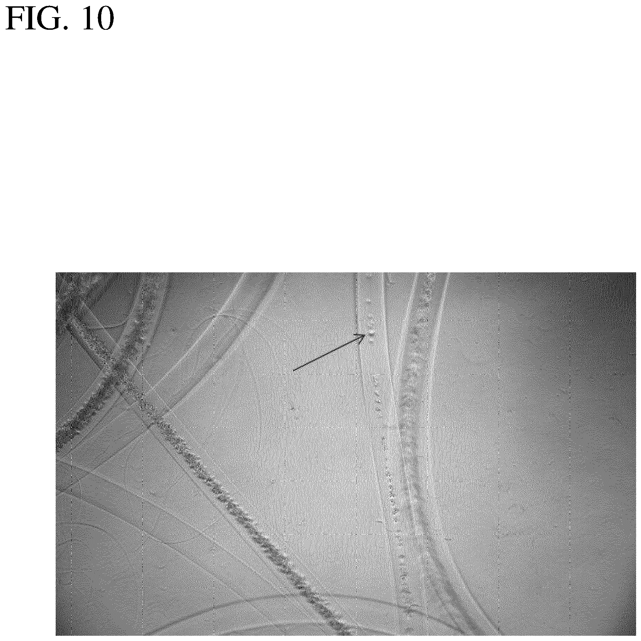

The evaluation item (1) indicates whether or not "confinement of cells at the end of the cell fiber" is achieved immediately after the cell fiber is transferred to the medium. In the first example, the confinement of cells was achieved in all samples, and a hydrogel region containing no cells was formed at the end of the cell fiber as shown in Fig. 4 of the present application. Further, as shown in Fig. 10, a region containing only hydrogel was present near the end of the cell fiber, and the cells were confined. The arrow added to the photomicrograph of Fig. 10 indicates the cell near the end of the cell fiber.

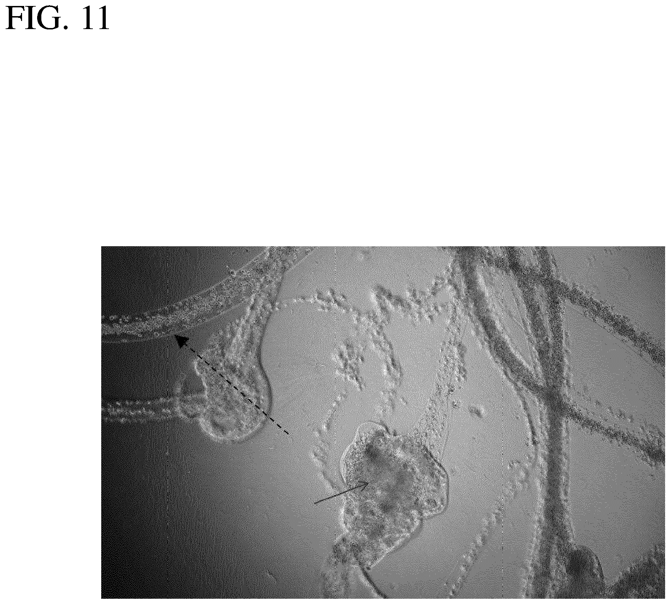

On the other hand, in the first reference example, as shown in Fig. 11, the cells at the end of the cell fiber were not confined but were mixed with the hydrogel to form a bump-like mass, and the cells leaked from the hydrogel (see a portion near the solid arrow in Fig. 11). In the first reference example, it was confirmed that such a situation occurred at the end of the cell fiber for all the samples.

The evaluation item (2) in Table 1 indicates whether or not the "prevention of mixture of cells with the hydrogel (gel formed by gelation of the second fluid)" is achieved immediately after the cell fibers in the first example and the first reference example are transferred to the medium. In the first example, mixture of cells with the hydrogel (hydrogel formed by gelation of the second fluid) could be prevented in all the samples (see also Fig. 10). That is, in the first example, the cells remain in the space inside the hydrogel (hydrogel formed by gelation of the second fluid) constituting the cell fiber.

On the other hand, in the first reference example, it was confirmed that the cells in the first fluid were mixed with the hydrogel in four samples out of five samples. It can be seen in Fig. 11 that cells are mixed with the hydrogel (portion near the outer surface of the hydrogel) on the outer shell of the cell fiber (see, for example, a portion near the dotted arrow in Fig. 11).

The evaluation item (3) in Table 1 indicates whether or not the cells leak while being cultured in the cell fiber. Specifically, the cell fibers were transferred to a medium to start culturing the cells, and after three days, the cell fibers were moved to a new well, and it was assessed whether or not the cells adhered to the inner surface of the new well the next day.

In the first example, it was found that cells did not adhere to the inner surface of the new well in four samples out of five samples, and the effect of suppressing cell leakage was relatively high even during cell culture.

On the other hand, in the first comparative example, it was found that the cells adhered to the inner surface of the new well in four samples out of five samples, and the cells leaked from the cell fiber during the culture of the cells.

As described above, it is found that, in the first example, leakage of cells from the end of the cell fiber can be suppressed at the initial stage of production of the cell fiber and during cell culture in the cell fiber, and an effect of confining the cells in at least the end of the cell fiber is high.

While the present invention has been described above by way of embodiments, the description and drawings that constitute a part of the disclosure should not be construed to limit the present invention. Various alternative embodiments, examples, and operational techniques would be obvious to a person skilled in the art from this disclosure. Accordingly, the technical scope of the present invention is defined only by the invention-specifying matters set forth in the claims appropriate from the above description.

For example, the cell fiber production methods in the first embodiment, the second embodiment, and the third embodiment described above include a step of delivering a cleaning fluid at a start stage or a stop stage. However, the step of delivering the cleaning fluid is not necessary, and may be performed as needed. Specifically, the step of delivering the cleaning fluid may not be performed at one or both of the start stage and the stop stage of the production. Even when cleaning is not performed at one or both of the start stage and the stop stage of the production, it is only sufficient that the first fluid, the second fluid, and/or the third fluid may be delivered in the order as described above. The cell fiber production system is preferably provided with the above-mentioned cleaning flow path 134 and the like so that cleaning can be performed at an appropriate timing such as maintenance even when the step of delivering the cleaning fluid is not performed at the start stage and the stop stage of the production.

It should also be noted that the various configurations described in the respective embodiments can be combined and exchanged wherever possible. For example, in the cell fiber production method, the process at the start stage of production described in the first embodiment and the process at the stop stage of production described in the second embodiment may be combined. Further, the process at the start stage of production described in the second embodiment and the process at the stop stage of production described in the third embodiment may be combined.

The flowcharts shown in Figs. 2, 5, and 8 may be manually or semi-manually performed by a person. For example, the setting of the cleaning pump 132, the first pump 112, the second pump 114, and the third pump 116 may be manually switched by a person. Alternatively, the setting conditions of the cleaning pump 132, the first pump 112, the second pump 114, and the third pump 116 may be automatically switched by a computer, for example, a computer mounted on the cell fiber production apparatus. In this case, the setting conditions, that is, the liquid delivery protocol, of various pumps may be preset by a program. In particular, the liquid delivery protocol at the start stage and/or the stop stage of the cell fiber production is preferably executed according to a predetermined program.

It should be noted that the scope of the present invention also includes a program for causing a computer to execute the above-mentioned cell fiber production method described with reference to the flowcharts shown in Figs. 2, 5, and 8. Such a computer program is stored in various types of non-transitory computer readable media and can be supplied to the computer. Non-transitory computer-readable media include various types of tangible storage media. Examples of non-transitory computer-readable media include a magnetic recording medium (for example, flexible disk, magnetic tape, and hard disk drive), a magneto-optical recording medium (for example, magneto-optical disk), a compact disk read only memory (CD-ROM), CD-R, CD-R/W, semiconductor memory (for example, mask ROM, programmable ROM (PROM), erasable PROM (EPROM), flash ROM, and random access memory (RAM)). The program may also be supplied to the computer by various types of transitory computer readable media. Examples of transitory computer-readable media include an electrical signal, an optical signal, and an electromagnetic wave. The transitory computer-readable medium can supply the program to the computer via a wired communication path such as an electric wire and an optical fiber, or a wireless communication path.

From the above-described embodiments, it can be understood that at least the following inventions are also described.

A cell fiber production system according to one aspect includes: a first flow path through which a first fluid containing a cell flows; a second flow path for allowing a second fluid for preparing a hydrogel to converge around the first fluid in the first flow path; and an ejection port through which at least the first fluid and the second fluid are discharged together. The cell fiber production system and/or method is configured such that, at a start stage of production of a cell fiber, the first fluid is delivered after the second fluid reaches at least a convergence point between the first flow path and the second flow path. Preferably, the cell fiber production system and/or method is configured such that, at the start stage of the production of the cell fiber, the first fluid is delivered after the second fluid reaches the ejection port.

Since the first fluid containing a cell is delivered after a flow of the second fluid, a front end of the cell fiber is sufficiently closed by the (gelled) second fluid. Thus, leakage of the cell in the cell fiber from the front end of the fiber during the production of the cell fiber can be prevented. When the cell leaks from the cell fiber, it is not always easy to find out the place where the cell leaks. It should be noted that the present inventor has discovered that the cell leaks from the front end or the rear end of the cell fiber depending on conditions, and arrived at the present invention.

The cell fiber production system and/or method according to another aspect is configured such that, at a stop stage of the production of the cell fiber, the delivery of the second fluid is stopped after the delivery of the first fluid is stopped.

In this aspect, the second fluid is delivered even after the delivery of the cell-containing first fluid is stopped, so that the rear end of the cell fiber is sufficiently closed by the (gelled) second fluid. This configuration makes it possible to prevent the cell from leaking from the rear end of the fiber during the production of the cell fiber.

According to another aspect, the cell fiber production system and/or method includes a third flow path for allowing a third fluid that gels the second fluid to join the first flow path on the downstream side with respect to the convergence point between the first flow path and the second flow path.

In this aspect, the second fluid for preparing a hydrogel can be partially or completely gelled with the third fluid before the first fluid containing a cell and the second fluid are ejected through the ejection port. Therefore, the cell fiber can be ejected through the ejection port in a more stable state, so that damage to the cell fiber can be suppressed. Further, when a mode in which the third fluid that gels the second fluid is converged in the nozzle is applied, there is an advantage that the thickness or the like of the cell fiber can be adjusted by adjusting the flow velocity of the third fluid.

According to another aspect, the cell fiber production system and/or method is configured such that, at the start stage of the production of the cell fiber, the first fluid is delivered after both the second fluid and the third fluid reach at least a convergence point between the third flow path and the first flow path. Preferably, the cell fiber production system and/or method is configured such that, at the start stage of the production of the cell fiber, the first fluid is delivered after both the second fluid and the third fluid reach the ejection port.

In this aspect, the first fluid is delivered after the second fluid and the third fluid join. In other words, the first fluid containing a cell is delivered after the front end of the cell fiber is partially or completely gelled. Thus, leakage of the cell in the cell fiber from the front end of the fiber during the production of the cell fiber can be prevented.

According to another aspect, the cell fiber production system and/or method is configured such that, at the start stage of the production of the cell fiber, the third fluid is delivered after the second fluid reaches a convergence point between the third flow path and the first flow path. Preferably, the cell fiber production system and/or method is configured such that, at the start stage of the production of the cell fiber, the third fluid is delivered after the second fluid reaches the ejection port.

In this aspect, the third fluid is delivered after the flow of the second fluid is stabilized, so that a laminar flow is easily formed by the second fluid and the third fluid. Thus, a possibility that the second fluid and the third fluid are mixed by a turbulent flow is reduced, and a possibility that the flow path in the nozzle 200 is clogged by the gelled second fluid can be reduced.

According to another aspect, the cell fiber production system and/or method is configured such that, at the stop stage of the production of the cell fiber, the delivery of the second fluid and the delivery of the third fluid are stopped after the delivery of the first fluid is stopped.

In this aspect, the second fluid and the third fluid converge and are ejected even at a stage where the delivery of the first fluid containing a cell is stopped. That is, the front end of the cell fiber is partially or completely gelled and the rear end of the cell fiber is closed by the hydrogel. This makes it possible to further prevent the cell from leaking from the rear end of the fiber.

According to another aspect, the cell fiber production system and/or method is configured such that, at the stop stage of the production of the cell fiber, the delivery of the second fluid is stopped after the delivery of the third fluid is stopped.

In this aspect, there is a period in which the second fluid flows even after the third fluid that gels the second fluid is stopped. Therefore, the second fluid expels the residue of the third fluid through the ejection port. Accordingly, it is possible to prevent the gelled fluid from staying in the flow path and causing clogging after the production of the cell fiber is stopped.

According to another aspect, the cell fiber production system and/or method further includes a cleaning flow path for allowing a cleaning fluid to flow into at least one of the first flow path and the second flow path.

In this aspect, at least a part of the first flow path and the second flow path can be cleaned through the cleaning flow path. Therefore, clogging of the flow path can be suppressed. In addition, this configuration provides an advantage that the flow path can be cleaned without disassembling the cell fiber production system.

According to another aspect, the cell fiber production system and/or method further includes a cleaning flow path for allowing a cleaning fluid to flow into the third flow path.

In this aspect, at least a part of the third flow path can be cleaned through the cleaning flow path. The third flow path is a flow path for the third fluid that gels the second fluid. If the second fluid accidentally flows into the third flow path, the second fluid will gel in the third flow path. This may cause clogging in the third flow path. Since the cleaning flow path communicates with the third flow path, such clogging in the third flow path can be eliminated by the cleaning fluid.

According to another aspect, the cell fiber production system and/or method further includes a liquid delivery pump for pumping the cleaning fluid from the cleaning flow path to the ejection port.

In this aspect, the cleaning fluid can flow from the cleaning flow path to the ejection port via the first flow path, the second flow path, and/or the third flow path by the liquid delivery pump. The third fluid gels the second fluid, and therefore, the flow path through which the third fluid passes may have clogging. In view of this, it is more preferable that the cleaning fluid passes through the convergence point between the third flow path and the first flow path and a portion on the downstream side with respect to the convergence point.

According to another aspect, the cell fiber production system and/or method is configured such that, at the start stage of the production of the cell fiber, the cleaning fluid is delivered at least before the delivery of the first fluid is started.

In this aspect, the production of the cell fiber can be started after the flow path in the nozzle is cleaned.

According to another aspect, the cell fiber production system and/or method is configured such that, at the stop stage of the production of the cell fiber, the cleaning fluid is delivered at least after the delivery of the first fluid is stopped.

In this aspect, the flow path in the nozzle can be cleaned at the stop stage of the production of the cell fiber, whereby an occurrence of clogging in the flow path after the production is stopped can be prevented.

According to another aspect, the cell fiber production system and/or method is configured such that, at the start stage of the production of the cell fiber, the cleaning fluid is delivered at least at or before a timing at which the delivery of the third fluid is started.

In this aspect, it is possible to start gelation of the second fluid after the flow path in the nozzle is cleaned. That is, the flow path in the nozzle can be cleaned before generation of the hydrogel.

According to another aspect, the cell fiber production system and/or method is configured such that, at the stop stage of the production of the cell fiber, the cleaning fluid is delivered at least at or after a timing at which the delivery of the third fluid is stopped.

In this aspect, it is possible to prevent the second fluid from flowing back into the third flow path due to the delivery of the cleaning fluid at the stop stage of the production. Further, even if the second fluid flows into the third flow path and gels in the third flow path, the gelled second fluid can be expelled through the ejection port by the delivery of the cleaning fluid. As a result, clogging in the nozzle 200 can be further suppressed.

According to another aspect, the cell fiber production system and/or method has a valve provided in the cleaning flow path. The valve may be, for example, an on-off valve or a one-way valve.

In this aspect, the cleaning flow path is closed by the valve at least while the cleaning fluid is not delivered, so that the first fluid, the second fluid, and/or the third fluid can be prevented from flowing back into the cleaning flow path.

According to another aspect, the cell fiber production system and/or method has a valve provided in the third flow path. The valve may be an on-off valve or a one-way valve.

In this aspect, it is possible to prevent the second fluid from flowing back into the third flow path. As a result, clogging due to gelation of the second fluid can be suppressed in a region of the third flow path in the vicinity of the convergence point between the first flow path and the third flow path.

According to another aspect, the cell fiber production system and/or method includes: a reservoir that receives an object ejected through the ejection port; and an adjustment mechanism for varying a distance between the reservoir and the ejection port.

In this aspect, the positional relationship between the ejection port and the liquid level in the reservoir can be freely changed during the production of the cell fiber.

According to another aspect, the cell fiber production system and/or method has a detector that can estimate or detect that the ejection port is located in a liquid in the reservoir.

In this aspect, it is possible to detect that the ejection port is located in the liquid in the reservoir prior to the production of the cell fiber. Thus, the adjustment mechanism can automatically change the distance between the reservoir and the ejection port so that the ejection port is located in the liquid in the reservoir.

According to another aspect, the cell fiber production system and/or method has a nozzle that includes the first flow path, the second flow path, the third flow path, and the ejection port, and the nozzle is replaceable.

In this aspect, when the flow path in the nozzle 200 is clogged, the clogging can be easily cleared by replacing the nozzle. Therefore, when the nozzle is configured to be replaceable, the cell fiber production system can be used for a long period of time.

According to another aspect, the nozzle for the cell fiber production system includes: a first flow path through which a first fluid containing a cell flows; a second flow path for allowing a second fluid for preparing a hydrogel to converge around the first fluid in the first flow path; a third flow path for allowing a third fluid that gels the second fluid to join the first flow path on a downstream side with respect to a convergence point between the first flow path and the second flow path; and an ejection port through which at least the first fluid, the second fluid, and the third fluid are discharged together. The nozzle is replaceable with respect to the cell fiber production system.

According to another aspect, the cell fiber includes a plurality of cells aligned along the fiber and a hydrogel that wraps around the cells in a cross section that intersects a direction of extension of the fiber. The hydrogel is closed at both ends of the fiber in the direction of extension of the fiber so as to confine the cells.

In this aspect, the cells are confined by the hydrogel at both the front end and the rear end of the fiber. Therefore, even if the cells are cultured in the fiber, leakage of the cells from the front end or the rear end of the fiber during the production of the cell fiber can be prevented.

- 100: Cell fiber production system
- 102: First storage unit
- 104: Second storage unit
- 106: Third storage unit
- 130: Container
- 132: Cleaning pump
- 134: Cleaning flow path
- 200: Nozzle
- 210: First inlet
- 212: First flow path
- 220: Second inlet
- 222: Second flow path
- 230: Third inlet
- 232: Third flow path
- 240: Ejection port
- 300: Reservoir
- 400: Stage

## Claims

1. A cell fiber production system comprising:
a first flow path through which a first fluid containing a cell flows;
a second flow path for allowing a second fluid for preparing a hydrogel to converge around the first fluid in the first flow path; and
an ejection port through which at least the first fluid and the second fluid are discharged together, wherein
the cell fiber production system is configured that, at a start stage of production of a cell fiber, the first fluid is delivered after the second fluid reaches at least a convergence point between the first flow path and the second flow path.

2. The cell fiber production system according to claim 1, wherein the cell fiber production system is configured that, at a stop stage of the production of the cell fiber, delivery of the second fluid is stopped after delivery of the first fluid is stopped.

3. The cell fiber production system according to claim 1 or 2, further comprising a third flow path for allowing a third fluid that gels the second fluid to join the first flow path on a downstream side with respect to the convergence point between the first flow path and the second flow path.

4. The cell fiber production system according to claim 3, wherein the cell fiber production system is configured that, at the start stage of the production of the cell fiber, the first fluid is delivered after both the second fluid and the third fluid reach at least a convergence point between the third flow path and the first flow path.

5. The cell fiber production system according to claim 4, wherein the cell fiber production system is configured that, at the start stage of the production of the cell fiber, the third fluid is delivered after the second fluid reaches the convergence point between the third flow path and the first flow path.

6. The cell fiber production system according to any one of claims 3 to 5, wherein the cell fiber production system is configured that, at a stop stage of the production of the cell fiber, delivery of the second fluid and delivery of the third fluid are stopped after delivery of the first fluid is stopped.

7. The cell fiber production system according to claim 6, wherein the cell fiber production system is configured that, at the stop stage of the production of the cell fiber, the delivery of the second fluid is stopped after the delivery of the third fluid is stopped.

8. The cell fiber production system according to any one of claims 1 to 7, further comprising a cleaning flow path for allowing a cleaning fluid to flow into at least one of the first flow path and the second flow path.

9. The cell fiber production system according to any one of claims 3 to 7, further comprising a cleaning flow path for allowing a cleaning fluid to flow into the third flow path.

10. The cell fiber production system according to claim 9, wherein the cell fiber production system is configured that, at the start stage of the production of the cell fiber, the cleaning fluid is delivered at least at or before a timing at which delivery of the third fluid is started.

11. The cell fiber production system according to claim 9 or 10, wherein the cell fiber production system is configured that, at a stop stage of the production of the cell fiber, the cleaning fluid is delivered at least at or after a timing at which delivery of the third fluid is stopped.

12. The cell fiber production system according to any one of claims 3 to 7 and 9 to 11, further comprising a valve provided in the third flow path.

13. The cell fiber production system according to any one of claims 1 to 12, further comprising:
a reservoir that receives an object ejected through the ejection port; and
an adjustment mechanism for varying a distance between the reservoir and the ejection port.

14. The cell fiber production system according to claim 13, further comprising a detector capable of estimating or detecting that the ejection port is located in a liquid in the reservoir.

15. The cell fiber production system according to any one of claims 3 to 7 and 9 to 12, further comprising a nozzle including the first flow path, the second flow path, the third flow path, and the ejection port, wherein
the nozzle is replaceable.

16. A cell fiber comprising:
a plurality of cells provided along the fiber and
a hydrogel that wraps around the cells in a cross section that intersects a direction of extension of the fiber, wherein
the hydrogel is closed at both ends in the direction of extension of the fiber so as to confine the cells.

17. The cell fiber according to claim 16, wherein a length of a region of the hydrogel containing no cells is larger than 1 mm at both ends of the fiber in the direction of extension of the fiber.

18. A cell fiber production method using a nozzle that includes
a first flow path through which a first fluid containing a cell flows,
a second flow path for allowing a second fluid for preparing a hydrogel to flow along a flow of the first fluid around the first fluid, and
an ejection port through which at least the first fluid and the second fluid are discharged together, the method comprising:
delivering the second fluid at a start stage of production of a cell fiber; and
delivering the first fluid after the second fluid reaches at least a convergence point between the first flow path and the second flow path.

19. The cell fiber production method according to claim 18, wherein
the nozzle comprises a third flow path for allowing a third fluid that gels the second fluid to join the first flow path on a downstream side with respect to the convergence point between the first flow path and the second flow path, and
the cell fiber production method includes delivering the first fluid after both the second fluid and the third fluid reach at least a convergence point between the third flow path and the first flow path at the start stage of the production of the cell fiber.

20. A program for making computer execute the cell fiber production method according to claim 18 or 19.
